(19) **Europäisches Patentamt European Patent Office Office européen des brevets**

(11) **EP 2 099 421 B1**

(12) **EUROPEAN PATENT SPECIFICATION**

(45) Date of publication and mention
of the grant of the patent:
**30.06.2010 Bulletin 2010/26**

(51) Int Cl.:
*A61K 8/34* (2006.01)     *A61K 8/35* (2006.01)
*A61K 8/36* (2006.01)     *A61Q 11/00* (2006.01)
*A23L 1/226* (2006.01)    *A23G 3/00* (2006.01)

(21) Application number: **07849288.1**

(22) Date of filing: **29.11.2007**

(86) International application number:
**PCT/IB2007/054844**

(87) International publication number:
**WO 2008/068683 (12.06.2008 Gazette 2008/24)**

(54) **ANTIMICROBIAL FLAVOURING COMPOSITION**

ANTIMIKROBIELLE GESCHMACKSZUSAMMENSETZUNG

COMPOSITION AROMATISANTE ANTIMICROBIENNE

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **01.12.2006 EP 06125259**

(43) Date of publication of application:
**16.09.2009 Bulletin 2009/38**

(73) Proprietor: **Firmenich SA
1211 Geneva 8 (CH)**

(72) Inventors:
• **BARRA, Jérôme
74160 Neydens (FR)**
• **SEYFRIED, Markus
74160 Lathoy (FR)**
• **TASHIRO, Hidemi
1206 Geneva (CH)**
• **TROCCAZ, Myriam
74160 St-Julien-en-Genevois (FR)**
• **BECCUCCI, Sabine
74800 ETEAUX (FR)**

(74) Representative: **Dale, Gavin Christopher
Firmenich SA
Route des Jeunes 1
P.O. Box 239
1211 Genève 8 (CH)**

(56) References cited:
**EP-A- 1 238 650      WO-A-2004/073671
WO-A-2005/104842**

• **DATABASE WPI Week 200517 Derwent
Publications Ltd., London, GB; AN 2005-155235
XP002430379 & JP 2005 015684 A (KAWASAKI K)
20 January 2005 (2005-01-20)**

**Description**

**Technical Field**

The present invention relates to a flavouring composition comprising two or more antimicrobial flavouring ingredients. The invention also relates to oral care or edible compositions comprising such a flavouring composition.

**Background and Prior Art**

It is well known that within the oral cavity, a large number and variety of bacteria exist. Many are known to have a detrimental effect on one or more of the gum, the teeth and the breath of an individual leading to, for instance, oral halitosis, periodontal disease and cariogenesis.

Bacteria are classified in terms of their response to the Gram staining protocol based on structural differences in their cell walls. The cell wall of Gram-positive bacteria containing higher level of peptidoglycans retain the crystal violet dye after an alcohol wash and will then appear blue/purple under the microscope. Gram-negative bacteria do not retain the crystal violet dye and will appear red/pink due to the addition of the counterstain Safranin.

About 90% of halitosis originates within the oral cavity. Bacterial formation of the odoriferous volatile sulphur compounds (VSC) hydrogen sulphide ($H_2S$) and methyl mercaptan ($CH_3SH$) within the oral cavity, especially in the tongue coating is the main cause of oral malodour. Oral halitosis is caused predominantly by Gram (-) anaerobic species such as *Fusobacterium nucleatum, Porphyromonas gingivalis, Prevotella intermedia. Klebsiella pneumoniae, Veillonella alcalescens* and *Bacteroides melaninogenicus/forsythus.* Periodontal disease is caused both by Gram (+) species such as *Actinomyces* and *Streptococci* and Gram (-) species such as *Spirochetes, Bacteroides, Selenomonas sputagena, Fusobacterium nucleatum, Porphyromonas gingivalis. Prevotella intermedia, Porphyromonas endodontalis.* Finally cariogenesis is caused by bacteria sugar metabolism dropping the pH below 5 thus releasing lactic acid, which dissolves calcium phosphate in the tooth. The bacterium primarily responsible for this is *Streptococcus mutans.*

Due to the multitude of bacterial species found in the oral cavity, there is a need for effective non-specific anti-microbial agents, with a broad spectrum of activity. Such agents will need to be compatible with a number of different preparations, including mouthwashes, mouthsprays, toothpastes and other dentifrices, chewing gum, candies and related functional products.

Since many oral care products are flavoured, it would be desirable that the flavouring ingredients also provide antibacterial effects since this would reduce the amount of additional antibacterial ingredients required.

The use of flavouring ingredients to provide oral care benefits has already been addressed. For instance, JP-A-2004/067530 (Kanebo Ltd) relates to removers for eliminating diet-independent halitosis due to periodontal diseases, visceral diseases, etc. The removers contain Labiatae plant extracts, lactoferrins, and optionally sugar alcohols, plant-derived polyphenols, acidulants, and linalool. The composition may be used in a chewing gum.

WO-A-2003/105794 (Takasago International Corporation) discloses a flavour and fragrance composition having an antibacterial activity effective against periodontal disease-causing bacteria and/or a halitosis-inhibition action capable of controlling production of volatile sulfides. The composition contains at least one substance selected from food fragrance materials such as hexaldehyde, caryophyllene alcohol, cinnamaldehyde, dihydroeugenol, farnesol, and grapefruit oil.

WO-A-99/51093 (Innocent Ltd) is directed to the use of a composition comprising a higher alcohol selected from 1-nonanol, 1-decanol and 1-undecanol, or mixtures thereof and taste-masking additives, as an oral anti-odour preparation. The composition is described for use in a toothpaste, a mouthwash, candies and other anti-odour preparations for oral use.

WO-A-2004/014348 (Michael Gurin) discloses a composition and method for functionalizing confectionery, chewing gum, oral care and beverage products by intensifying the flavor using ingredients comprised of flavor potentiators, enhancers, and amplifiers. The composition further comprises oral care actives selected to control halitosis and dental plaque utilizing polyphenols and enzymes whose activity levels are protected by stabilization methods.

JP-A-2003/026527 (Lion Corporation) relates to the active agents 2,6-dimethyl-3,7-octadiene-2,6-diol, 2,6-dimethyl-1,7-octadiene-3,6-diol, 3-acetoxy-1-p-menthane, 8-acetoamino-1-p-menthane, 11-hydroxy-8-eudesmene, and extracts of orange (Zanthoxylum) that contains C16 or higher unsaturated fatty acids and C6 or higher unsaturated aldehydes, for controlling *F. nucleatum* bacteria in the mouth without apparently affecting beneficial microorganisms in humans.

EP 1 238 650 (Takasago) relates to antimicrobial flavour compositions and oral care compositions or foods containing antimicrobial flavour compositions. The compositions are described as comprising diverse flavouring antimicrobial compounds. Moreover, several of these materials are characterized by a MIC below 1000 ppm against microorganisms responsible of oral halitosis. However, 3,4-dimethylphenol, as well as other ingredients like anethole, hydrocinnamic aldehyde, isoeugenol extra and 2-methyl hexanoic acid are not mentioned.

WO 2005/104842 (MICAP) discloses antimicrobial compounds in the medical field. 3,4-dimethylphenol is disclosed in a long list of carriers for antimicrobial compounds, but is not described as an antimicrobial itself.

It would also be desirable to provide antibacterial flavouring ingredients which are effective at very low doses since flavouring ingredients cannot normally be used in high amounts without providing an overpowering flavour which is often undesirable to the consumer.

It is an object of the present invention to provide one or more of the abovementioned benefits and/or to address one or more of the abovementioned problems.

## Summary of the Invention

Accordingly, the present invention provides a flavouring composition comprising an antimicrobial key and optionally at least one flavouring ingredient of current use, wherein the antimicrobial key comprises 3,4-dimethylphenol together with one or more antimicrobial flavour ingredients each having a minimum inhibitory concentration of 1000 parts per million or less against two or more strains selected from *Fusobacterium nucleatum, Fusobacterium sp., Porphyromonas gingivalis, Prevotella intermedia, Klebsiella pneumoniae, Veillonella alcalescens, Bacteroides melaninogenicus/forsythus, Selenomonas sputagena, Porphyromonas endodontalis, Prevotella melaninogenica* and *Streptococcus mutans.*

In a further aspect, the invention provides an oral care product comprising the antimicrobial flavouring composition.

In yet a further aspect, the invention provides a confectionary product comprising the antimicrobial flavouring composition.

In yet another aspect, the invention provides a beverage comprising the antimicrobial flavouring composition.

## Detailed Description of the Invention

The term "antimicrobial" is used to mean effective to kill, inhibit or inactivate at least a proportion of one or more strains of bacteria.

The minimum inhibitory concentration (referred to herein as "MIC") is used to mean the concentration, in parts per million, of antimicrobial flavouring ingredients sufficient to lead to a complete inhibition of growth of a strain of bacteria.

The bacterial contact time (referred to herein as "BCT") is defined as a measure of the efficacy with which a product solution, at a defined concentration and/or after a defined contact time, will kill a given type of bacteria introduced into that solution.

Flavouring compositions are a carefully balanced mixture of numerous different flavouring ingredients in which, to achieve a desired flavour, there are often very small amounts of individual ingredients. Therefore, if a flavouring ingredient is to be considered as an effective antimicrobial flavouring ingredient, it must be able to inhibit bacterial growth even at very low concentrations.

The present inventors have surprisingly found certain flavouring ingredients having an MIC of 1000 or less which provide excellent antimicrobial characteristics to the flavouring composition in which they are present. Particularly effective are compositions in which 3,4-dimethylphenol is present.

Thus the term "effective" when used to describe the MIC of the antimicrobial flavouring ingredient denotes an MIC of 1000 or less.

### Antimicrobial Key

3,4-dimethylphenol is mixed with at least one flavouring ingredient having the desired MIC and preferably the desired BCT to form an antimicrobial flavour key. This key can then be added to a standard flavour composition to boost its antimicrobial effect without adversely affecting the desired flavour characteristics.

The antibacterial key may advantageously comprise 3,4-dimethylphenol together with at least 2 flavours having the desired MIC and preferably the desired BCT.

It is beneficial, in the context of the present invention, that the antimicrobial key comprises more than one antimicrobial ingredient since it is important that the key not only provides antimicrobial benefits but also has a balanced flavour profile. For instance, if the key contains only one ingredient, then the flavour is likely to be unbalanced whereas when two or more flavours are present, less of each ingredient is required and the inherent risk of unbalanced, overpowering flavours is diminished.

It has been found that, in order to be effective at inhibiting or reducing microbial activity in the oral cavity, the antimicrobial key can be present in an amount as low as 1 to 20% by weight based on the total weight of the flavour composition. Surprisingly low levels such as I to 15% by weight, or even 1 to 12% by weight are also found to be effective.

Since the flavour composition is typically incorporated into the final product at a level of 0.05 to 1% by weight, more preferably 0.06 to 0.5% by weight, most preferably 0.07 to 0.3% by weight, the antimicrobial key can surprisingly be present in very small amounts and yet still remain effective. For instance, the antibacterial key may be present in an amount of from 0.001 to 0.5%, more preferably 0.003 to 0.3, most preferably 0.008 to 0.05% by weight, based on the total weight of the final product.

Minimum Inhibitory Concentration

The antimicrobial flavouring ingredients used in the composition of the present invention have an MIC of 1000 or less, more preferably 900 or less, even more preferably 850 or less, most preferably 800 or less against two or more strains selected from *Fusobacterium nucleatum, Fusobacterium sp., Porphyromonas gingivalis, Prevotella intermedia, Klebsiella pneumoniae, Veillonella alcalescens, Bacteroides melaninogenicus/forsythus, Selenomonas sputagena, Porphyromonas endodontalis, Prevotella melaninogenica* and *Streptococcus mutans.*
More preferably at least one of the antimicrobial flavouring ingredients has the abovementioned MIC against 3 or more, or even 4 or more of the abovementioned strains.
It is also preferred that at least one ingredient has an MIC of 1000 or less, more preferably 900 or less, even more preferably 850 or less, most preferably 800 or less against two or more strains selected from *Fusobacterium sp., Klebsiella pneumoniae, Veillonella alcalescens, Bacteroides melaninogenicus/forsythus, Selenomonas sputagena, Porphyromonas endodontalis* and *Prevotella melaninogenica*.
There are various well known tests already in existence for determining MIC. See for instance, Mann CM, Markham JL, A New Method For Determining the Minimum Inhibitory Concentration of Essential Oils, J. of Applied Microbiology, 1998; 84: 538-544.
For the purposes of the present invention, MIC is measured as follows:
A dilution series of 16 concentrations ranging from 150 ppm to 10000 ppm and including at least 500, 800, 850, 900 and 1000ppm of the active to be tested is prepared by dissolving the active in an appropriate solvent. The dilution series is then transferred to 96-well microtitre plates that have been supplemented with a suitable aqueous growth media. The plates are inoculated with a microbial test strain of interest and incubated under shaking at 37°C under anaerobic conditions. After 24 hours, the growth of the test strain is measured as a function of optical density.

Bacterial Contact Time

In addition to having a very low minimum inhibitory concentration, it is further preferred that the antimicrobial flavouring ingredient has the capacity to kill given bacteria in a short time.
This is particularly important since the antimicrobial flavouring ingredient will be present in an oral care composition which may only be in contact with the bacteria in the mouth for a short period, typically 60 seconds or less.
The time taken for an ingredient to kill a bacterial strain can be measured in vitro using the bacterial contact time, defined below.
Bacterial kill is measured by sampling the mixture at short time interval after mixing, stopping the kill action, and then growing the remaining viable bacteria, which can be counted by optical density measurement. The measure of bacterial kill is in the form of the time taken to achieve a given level of bacterial kill, usually 99.9% or log reduction of bacterial counts after a defined contact time.
In the present invention, BCT was measured by firstly diluting a sample of the flavouring ingredient to 1% in 25% ethanol and then applying between 0.01% to 1% dosage. The log reduction in bacterial count was then measured after 80 seconds.
It is preferred that at least one of the antimicrobial flavouring ingredients has a BCT log reduction of 2 or more, more preferably 2.5 or more, most preferably 3 or more against cariogenic bacteria S. *mutans* and/or a log reduction of 1 or more, more preferably 2 or more against anaerobic pathogenic strains *Fusobacterium nucleatum, Porphyromonas gingivalis, Prevotella intermedia, Klebsiella pneumoniae, Veillonella alcalescens* and *Bacteroides melaninogenicers/forsythus* after 80 second contact time.
It is also preferred that at least one of the antimicrobial flavouring ingredients has a BCT log reduction of 1 or more, more preferably 2 or more against anaerobic pathogenic strains *Prevotella intermedia, Klebsiella pneumoniae, Veillonella alcalescens* and *Bacteroides melaninogenicus/forsythus* after 80 second contact time.
The antibacterial flavouring ingredients may be used to replace, in whole or in part, conventional antibacterial materials used in the flavoured products of interest, without diminishing the antibacterial properties of the product. By "conventional antibacterial materials", it is meant antibacterial materials, which are not associated with any flavouring characteristics and ofter have an undesirable aroma wich must then be masked using known flavour ingredients. For example, mouthwashes conventionally include antibacterial agents such as triclosan (2', 4, 4'-trichloro-2-hydroxy-diphenyl ether), which is commercially available e.g. under the Trade Mark Irgasan DP 300, and trichlorocarbanalide (TCC) (also known as triclocarban). Triclosan is a broad spectrum antimicrobial agent which is known to provide excellent bacteriostatic activity at low concentrations against both Gram positive bacteria and Gram negative bacteria. Triclosan is the antibacterial commonly used in antibacterial soaps. TCC is effective only against Gram positive bacteria. By incorporating one or more antibacterial flavouring materials into such products, the levels of triclosan and/or TCC may be reduced, with consequent cost savings, without reducing the antibacterial efficacy of the product.

Alternatively, the antibacterial flavouring compositions of the invention can be used in combination with conventional antimicrobial agents, in which case this has been found to provide a synergistic effect reinforcing the antimicrobial character of such ingredients.

By its anti-microbial and flavouring properties, a flavouring composition prepared according to the invention is equally suitable for applications in all types of oral care and/or confectionary products.

The flavouring compositions of the invention may contain other constituents which have a positive or synergistic effect on the antimicrobial activity of the ingredients.

Testing has demonstrated that, in addition to 3,4-dimethylphenol, the following flavouring ingredients have the desired MIC characteristics required by the present invention:

acetyl cedrene, anethole ((e)-1-methoxy-4-(l-propenyl)benzene), Bacdanol® (2-ethyl-4-(2,2,3-trimethyl-3-cyclopenten-1-yl)-2-buten-1-ol), Brahmanol® (2-methyl-4-(2,2,3-trimethyl-3-cyclopenten-1-yl)-1-butanol), cashmerane, cinnamic aldehyde, dimethyl acetal, cyclohexadecen-1-one, cyclopentadecanolide, cyclopentadecanone, decen-1-ol, dihydrofamesol, cedrol, cedryl acetate, dodecanal, ethylenedodecanedioate, hydrocinnamic aldehyde, isobutyl quinoline, isocamphylcyclohexanol, isoeugenol extra, levosandol, Lilial® lg (3-(4-tert-butylphenyl)-2-methylpropanal), menthoxypropane 1,2 diol, methyl atrarate (methyl-2,4-dihydroxy-3,6-dimethyl-benzoate), methyl decanal, methyl sandeflor, methyl undecanal, nerolidol (3,7,11-trimethyl-1,6,10-dodecatrien-3-ol), nonanol, nootkatone ((+)-(4r)-4,4a,5,6,7,8-hexahydro-6-isopropenyl-4,4a-dimethyl-2(3h)-naphthalenone), 2-methylhexanoic acid, o-methoxycinnamic aldehyde, perilla alcohol (1,8-p-menthadien-7-ol), Phantolid® (1-(1,1,2,3,3,6-hexamethyl-5-indanyl)-1-ethanone), phenylacetaldehyde, Sandalore® ((+)-3-methyl-5-(2,2,3-trimethyl-3-cyclopenten-1-yl)-2-pentanol), santalinol ((e)-2-methyl-4-(2,2,3-trimethyl-3-cyclopenten-1-yl)-2-buten-1-ol), trans-2-undecenal, undecanal, undecen-1-ol, 3-dodecenal, isopropyl myristate and helional.

This list is not exhaustive and the skilled person will, following the protocol detailed above for establishing MIC, readily ascertain whether a flavouring compound has the desired antimicrobial characteristics.

The finding that certain flavouring ingredients have excellent antimicrobial characteristics is very surprising. In particular, 3,4-dimethylphenol is reviewed in Amicbase (A. Pauli, ReviewScience), a CD-ROM database about growth inhibitory properties of organic compounds towards microorganisms. In this document, minimum inhibitory concentrations of 3,4-dimethylphenol against six bacterial strains are given (see table below). With the exception of *Mycobacterium tuberculosis,* a human pathogen not related to any of strains relevant for the purposes of the present invention, MIC values ranging from 2000 to 4000 ppm have been described. This is significantly less activity than we have discovered in the present invention.

Table 1

| Test strain | MIC [ppm] |
| --- | --- |
| *Mycobacterium tuberculosis subsp. tuberculosis* | 20 |
| *Staphylococcus aureus* | 2000 |
| *Streptococcus sp.* | 2000 |
| *Eschericia coli* | 4000 |
| *Proteus mirabilis* | 4000 |
| *Pseudomonas aeruginosa* | 4000 |

Furthermore, as described in Nucleotide Sequence and Function Analysis of the Complete Phenol/3,4-dimethylphenolcatabolic Pathway of *Pseudomonas sp.* Strain CF600, Shingler, Powlowski, Marklund, Journal of Bacteriology, Feb 1992, 711-724, some bacteria can even use 3,4-dimethylphenol as a carbon source and, in such cases, the presence of 3,4-dimethylphenol stimulates growth instead of inhibiting it. Therefore, the strong antimicrobial activity of 3,4-dimethylphenol could not have been anticipated.

The presence of 3,4-dimethylphenol in the compositions of the present invention, confers to said compositions a strong antimicrobial activity.

Preferably, 3,4-dimethylphenol is present in an amount of from 1 to 20% by weight, more preferably 2 to 15%, most preferably 6 to 12% by weight, based on the total weight of the key.

Preferred additional antimicrobial flavouring ingredients include anethole, hydrocinnamic aldehyde, acetyl cedrene, cyclopentadecanone, nonanol, isoeugenol extra, 2-methylhexanoic acid, and Brahmanol ®.

If the key comprises cyclopentadecanone, it is preferably present in an amount of from 1 to 10% by weight, more preferably 2 to 7%, most preferably 3 to 6%, based on the total weight of the key.

If the key comprises isoeugenol extra, it is preferably present in an amount of from 1 to 99% by weight, more preferably 40 to 97%, most preferably 80 to 94%, based on the total weight of the key.

If the key comprises acetyl cedrene, it is preferably present in an amount of from 1 to 45% by weight, more preferably 5 to 35%, most preferably 15 to 30%, based on the total weight of the key.

If the key comprises 2-methylhexanoic acid, it is preferably present in an amount of from 1 to 99% by weight, more preferably 60 to 98%, most preferably 70 to 95%, based on the total weight of the key.

If the key comprises Brahmanol ®, it is preferably present in an amount of from 1 to 50% by weight, more preferably 1 to 20%, most preferably 1 to 10%, based on the total weight of the key.

Even more preferably the additional antimicrobial flavouring ingredient is selected from anethole, hydrocinnamic aldehyde, isoeugenol extra and 2-methylhexanoic acid.

Whilst the choice of ingredients will be dictated by an MIC of 1000 or less and preferably a BCT log count of 2 or more against cariogenic bacteria S. *mutans* and a log reduction of 1 or more against anaerobic pathogenic strains after 80 second contact time, they should of course be capable of providing a flavouring effect in the composition or should, at least, be flavour neutral so that they can easily be incorporated into the end products mentioned herein.

Other Flavouring Ingredients

The antimicrobial key is, optionally together with at least one flavouring ingredient of current use, typically incorporated into a foodstuff or oral care product.

The term "flavouring ingredients of current use" is used to mean ingredients well known to the person in the flavouring art and having the necessary olfactive characteristics. The contribution of such flavouring ingredients to the final flavour will be substantially or even purely for taste purposes and the choice of these ingredients will depend on the nature of the product to be flavoured.

Such flavouring ingredients are compounds which, due to their high volatility or vapour pressure, reach olfactory receptors in the nose before and during the eating and drinking.

For the purpose of the present invention, a flavour is a compound that is characterised by a vapour pressure of ≥ 0.01 Pa at 25°C. Most flavours have a vapour pressure above this value, while lipids, such as animal fats, oleic acid, etc, generally have a vapour pressure lower than that. For the purpose of the present invention and for the sake of convenience, the vapour pressure is determined by calculation. Accordingly, the method disclosed in "EPI suite"; 2000 U.S. Environmental Protection Agency, is used to determine the concrete value of the vapour pressure of a specific compound or component of the ingredient. This software is freely available and is based on average values of vapour pressures obtained by various methods of different scientists. Typically the flavour ingredient will have an MIC above 1000ppm and so will not have significant antimicrobial properties.

Many suitable flavouring ingredients of current use are listed in reference texts such as in the book by S. Arctander, Perfume and Flavour Chemicals, 1969, Montclair, New Jersey, USA, or its more recent versions, or in other works of similar nature such as Fenaroli's Handbook of Flavour Ingredients, 1975, CRC Press or Synthetic Food Adjuncts, 1947, by M.B. Jacobs, van Nostrand Co., Inc.

The antimicrobial ingredients of the key, the key as a whole or the flavouring composition comprising the key can be incorporated in any delivery system or may be encapsulated following standard procedures. Thus, the flavouring composition may be encapsulated or may comprise an encapsulated component, the encapsulated component being one or more of the ingredients of the key or even the entire key.

Encapsulation of the key or of the flavouring composition comprising the key is advantageous because it enables the rapid, simultaneous release of the antimicrobial key components rather than a slower, longer release and is desirable where delivery of a concentrated dose is required. It is believed that this may further improve the antimicrobial efficiency of the key.

The skilled person in the art is well aware of a variety of encapsulation systems which are suitable for this purpose, the following being preferred for their properties of providing very good barriers to oxidation.

A first preferred encapsulating system is a glassy matrix within which the flavouring composition or the antibacterial key is held. More preferably the encapsulation system is a glassy carbohydrate matrix. The carbohydrate matrix ingredient preferably comprises a sugar derivative, more preferably maltodextrin.

Particularly preferred maltodextrins are those with a DE of from 10 to 30, more preferably from 15 to 25, most preferably from 17 to 19.

Typically, the flavouring composition or the antibacterial key is admixed with a carbohydrate matrix material and an appropriate amount of a plasticizer, such as water, the mixture is heated within a screw extruder to a temperature above the glass transition temperature of the matrix material so as to form a molten mass capable of being extruded through a die and then the molten mass is extruded using established processes, such as described in the prior art. See, for instance, patent application WO 00/25606, published May 11, 2002 or WO 01/17372, published March 15, 2001, and the documents cited therein.

If desired, further carbohydrate matrix components may be present to improve yet further the antioxidant barrier properties.

Other suitable encapsulation systems are described in, for examples, US 4,610, 890 or US 4,707,367.

End Products

Typical foodstuffs in which the composition can be used include confectionary, e.g. sweets such as hard-boiled candies, pastilles or lozenges for sucking and are thus held in the oral cavity for an extended period, for example up to 1 minute, whilst being allowed to dissolve.

Suitable oral care products include mouthwashes, mouthrinses, chewing gums and toothpaste.

The antimicrobial flavouring composition may also be incorporated into beverages. Typical beverages into which the antimicrobial flavouring composition can be incorporated include, for example, carbonated soft drink, functional soft drinks, juices and nectars, hot drinks, powdered soft drinks, alcoholic drinks.

The antimicrobial flavour compositions of the invention can also advantageously be used in the field of pet food. As non-limiting example of pet food products we can mention chewy bones for dogs.

## **Examples**

The invention will now be illustrated by way of the following examples. All amounts are percentage by weight unless otherwise stated.

Example 1

Determination of minimum inhibitory concentrations

The MIC of various flavouring ingredients was evaluated against *Streptococcus mutans* DSM 6178, *Fusobacterium nucleatum* DSM 20482, *Porphyromonas gingivalis* DSM 20709 and *Prevotella melaninogenica*.

The inoculum for the MIC assay was prepared by streaking out frozen material from freezer stocks at -80°C onto agar plates (Schaedler medium containing 5 % sheep blood (Biomerieux, Switzerland)), which were grown at 37 °C for 24 h in a plastic jar under anaerobic conditions in the presence of $CO_2$ in the case of *S. mutans,* or for 48 h in the absence of $CO_2$ in the case of the remaining 3 strains. Fresh single colonies were transferred into liquid Wilkins-Chalgren medium (Oxoid, UK, 25 ml in a 100 ml shake flask) and incubated under the same conditions as above for *S. mutans,* and for 72 h in the case of the remaining 3 strains.

Cultures that had reached an optical density (measured at a wavelength of 600 nm) of between 0.8 and 1.0 were washed once in fresh medium (5,000 rpm, 5 min at 4 °C in 50 ml Falcon tubes) and then diluted 50-fold in liquid Wilkins-Chalgren medium either with 0.15 % agar for *S. mutans* and *P. gingivalis* or without agar for *F. nucleatum* and P. *melaninogenica.* The inoculum was then used immediately in the test.

Semi-automated microdilution assays were performed using a Tomtec Quadra 3 dispensing station (Tomtec, USA). In a first step, assay plates (sterile 96-well flat-bottom polystyrene microtiter plates, Nunc, Denmark) were filled with 100 $\mu$l of the appropriate strain-specific medium (as used for the final 50-fold dilution, see above).

Then the five test materials were dissolved in ethanol (for testing against *S. mutans* and *P. gingivalis*) or in DMSO (for testing against *F. nucleatum* and *P. melaninogenica*). Line H of a sterile 1 ml round-bottom polystyrene deepwell plate (Nunc, Denmark) was filled with 525 $\mu$l of sterile-filtered stocks of 5 test materials dissolved in the appropriate solvent at two concentrations each (5 and 20 % w/v). The remaining 2 wells of line H were filled with the same volume of solvent (for positive control, i.e. growth in the absence of raw material, and negative control, i.e. sterility in the absence of inoculum, respectively). Lines A to G were filled with 175 $\mu$l of sterile solvent by the dispensing station. 1.5-fold dilutions of the stocks were then prepared by transfer of 350 $\mu$l from one lane to the next lane and mixing in between, until line A was reached. After this, 10 $\mu$l aliquots each were transferred from the deepwell plate to 3 assay plates prepared in the first step and the assay plates were filled with 90 $\mu$l of inoculum (one of the four test strains), except for column no. 12 (sterility control).

The assay plates were then sealed and incubated under shaking (160 rpm) at 37°C in a plastic jar for 24 h in the presence of $CO_2$ for *S. mutans* and for 72 h in the absence of $CO_2$ for the other three test strains as described earlier for agar plates.

Growth was then determined by measuring the optical density at a wavelength of 600 nm in a microtiter plate reader (Ultramark, BioRad, USA) after removal of the seal.

Growth was compared to the negative control, and the lowest among the 16 concentrations of each raw material that had lead to a complete inhibition of growth of the test strain represented the minimum inhibitory concentration (MIC value). Results were only taken into account if all positive controls had shown expected levels of growth in the absence of raw materials (only pure solvent added) and negative controls had remained sterile.

Since for each series of raw materials per microtiter plate tests were done in triplicate, final results were calculated as the average of 3 MIC values.

The MIC results (ppm) for a selection of antibacterial flavouring ingredients are given in the following table.

Table 2

| Composition | *Streptococcus mutans* | *Prevotella melaninogenica* | *Fusobacterium nucleatum* | *Porphyromonas gingivalis* |
|---|---|---|---|---|
| 3,4-dimethylphenol | 150 or less | 590 | 500 | 150 or less |
| Brahmanol ® | 250 | Not tested | 250 | 150 or less |
| Methylatrarate | 1000 | Not tested | 150 or less | 150 or less |
| Cinnamic aldehyde | 250 | Not tested | 500 | 150 or less |
| Acetyl cedrene | 250 | Not tested | 500 | 150 or less |
| 2-Methylhexanoic acid | 150 or less | 1500 | 500 | 1300 |
| Cyclopenta-decanone | 1000 | Not tested | 1000 | 150 or less |

Example 2

Preparation of antimicrobial keys

Antimicrobial keys having the compositions given in table 3 were prepared by mixing the ingredients until a homogeneous solution formed.

Table 3

| | Key | | | | |
|---|---|---|---|---|---|
| Ingredient | 1 | 2 | 3 | 4 | 5 |
| Brahmanol ® | - | 5.2 | - | 1 | - |
| DMP(1) | 4.1 | 12 | 8 | 20 | 10 |
| Methylatrarate | - | - | - | 9 | - |
| Nonanol | 2.5 | 25 | - | 15 | - |
| 2-Methylhexanoic acid | 93.4 | 7.8 | 91 | 20 | 90 |
| Acetyl cedrene | - | 23 | - | 20 | - |
| Natactone dextro(2) | | | 1 | | |
| Cyclopenta-decanone | - | 27 | - | 15 | - |
| (1) 3,4-dimethylphenol<br>(2) (+)-(3S,3AS,6R,7AR)-perhydro-3,6-dimethyl-benzo[B]furan-2-one (10% PG) | | | | | |

Example 3

Preparation of a flavouring composition comprising antimicrobial key

The flavouring composition having the formulation given in the table below was prepared by mixing the ingredient together with the antimicrobial key until a homogenous solution formed.

Table 4

| Ingredient | Flavour Composition |
|---|---|
| Menthol | 90 |
| Key 3 | 10 |

Example 4

Toothpaste comprising the antimicrobial keys

Oral care compositions were then prepared using the antimicrobial keys as follows:

Toothpastes having the formulations given in the table below were prepared by mixing the water and xylitol to form an aqueous solution, adding the glycerin (in which the carrageenan had previously been dispersed) followed by the sorbitol and then mixing for a period of about 20 minutes to hydrate the gum. The mixture was then introduced into a mixer under a vacuum and the remaining ingredients were added to the vacuum mixer. Mixing under vacuum was performed for a period of about 15 minutes, and the final mixture was then placed in a standard tube.

Table 5

| Ingredient | Toothpaste 1 | Toothpaste 2 |
|---|---|---|
| Vegetable glycerin | 10 | 30 |
| Sorbitol | 20 | 10 |
| Hydrated silica | 20 | 15 |
| Water | 20 | 10 |
| Xylitol | 17 | 30 |
| Carrageenan | 3 | 2 |
| SLS (1) | 3 | 1 |
| Titanium dioxide | 2 | 1 |
| Flavour composition | 3 | 0.1 |
| (1) sodium lauryl sulphate | | |

Example 5

Mouthwash comprising the antimicrobial keys

Mouthwashes having the formulations given in the table below were prepared by dissolving the solid ingredients in the water, adding the antibacterial key and stirring to ensure a homogeneous product.

Table 6

| Ingredient | Mouthwash 1 | Mouthwash 2 |
|---|---|---|
| Chlorhexidine gluconate | 0.1 | 0.5 |
| Xylitol | 3.0 | 2.0 |
| Ethyl alcohol | 5.0 | 3.5 |

(continued)

| Ingredient | Mouthwash 1 | Mouthwash 2 |
|---|---|---|
| Distilled water | 90.0 | 90.0 |
| Flavour composition | 1.9 | 4.0 |

Examples 5a and 5b

Hard boiled candies comprising the antimicrobial keys

(5a) A hard boiled candy having the formulation given in the table below was prepared by cooking together the ingredients (apart from the antimicrobial key) at 140°C for about 20 minutes until all components were thoroughly blended. The antimicrobial key was then blended in, after which the mass was poured onto a slab, kneaded and cooled. The candy mass, at 70°C, was shaped according to standard processes using drop rollers.

Table 7

| Ingredient | Sample 1 |
|---|---|
| Sucrose | 57.0 |
| Corn Syrup DE 40 | 35.0 |
| Colouring | 0.05 |
| Flavouring composition | 0.6 |
| Residual water | To 100 |

(5b) A sugar free hard boiled candy was prepared by mixing the isomalt with water, bringing the mixture to 160°C, then cooling the mixture quickly to 135°C, followed by adding 2ml of HIS solution (10wt% aspartame, 5wt% acesulfame potassium and 85% water), optionally together with 3ml of citric acid solution (50wt% citric acid solids, 50wt% water). The flavouring composition was then added in an amount of 0.1wt% based on the total weight of candy and the liquid mixture was poured into empty ejector pin moulds and allowed to solidify.

Example 6

Chewing gum comprising the antimicrobial keys

Table 8

| Ingredient | Sample 1 |
|---|---|
| Crystalline sorbitol (P56) | 54.8 |
| Acesulfame potassium | 0.1 |
| Aspartame | 0.1 |
| Sierra gum base (ex Cafosa) | 22 |
| Humectant syrup (Lycasin ® 80/55, ex Roquette) | 12 |
| Sorbit ® (78% solids) | 6 |
| Glycerin (99% solids) | 4 |
| Flavour composition | 0.1 |

The sorbitol, acesulfame potassium and aspartame were blended together in a Turbula blender. Half of the powdered blend was then mixed with the gum base which had been pre-warmed in a sigma-blade mixer at 50 to 55°C for 5 minutes. The remaining powder, the humectant, the Sorbit® solids and the glycerin were added to the gum base and mixed for 7 minutes. Finally the antibacterial flavour composition was added.

Example 7

Bacterial Contact Time test

Three samples were prepared with a regular flavour either alone or combined with an antimicrobial flavour key of the invention. The composition of the samples is summarized in the following table:

Table 9

| Sample N° | Antimicrobial Key |
|-----------|-------------------|
| 2 | None |
| 3 | 6 |
| 4 | 7 |

As regular flavour there was used a flavouring composition of the citrus type containing only flavouring ingredients that are not suitable ingredients for the antimicrobial key according to the present invention (i.e. that have a MIC above 1000 against the bacterial strains cited in the present application).

Antimicrobial flavour keys 6 and 7 were prepared by admixing the following ingredients in the following proportions:

Table 10

| Ingredients | Key 6 | Key 7 |
|-------------|-------|-------|
| 2-methylhexanoic acid | 930 | 910 |
| 3,4-dimethylphenol | 60 | 80 |
| Natactone dextro (1) | 10 | 10 |
| (1) (+)-(3S,3AS,6R,7AR)-perhydro-3,6-dimethyl-benzo[B]furan-2-one (10% PG) | | |

A pure culture of *Fusabacterium nucleatum* was grown in anaerobic conditions during 72 hours in liquid wilkins-chalgren medium (Biomérieux).

An aliquot of $10^7$ cells was added to 100 μl of each sample in a 96-microtiter plate and mixed thoroughly during 30 s. This was repeated eleven times for each of the samples. A control was performed in a well with $10^7$ cells that were not contacted with any sample.

The sterility of the environment was checked with a well containing only the bacterial growth media.

The plate was let stand during a bacterial contact time of 80 s. Any antimicrobial activity of the samples was then neutralized by a rapid 1000 times dilution in liquid broth media. Serial dilutions (100μl into 100μl of growth media) were performed until extinction of bacteria.

Determination of bacterial growth and bacterial counts were carried out by optical density at 600nm after 72 hours incubation in anaerobic conditions. Wells with no growth were taken as having zero surviving bacteria. Therefore the first well with growth was considered to contain at least one viable cell (log10 value of zero) before the 72 hours incubation. Counts of bacteria into the control wells were used to deduce bacterial percentage reduction (or bacterial log reduction) for each flavour:

$$\text{Bacterial Log reduction} = \text{Log} \left[ (\text{Average bacterial count in control wells}) / (\text{Average bacterial count in test samples}) \right]$$

The following table summarizes the results of the assay. No antimicrobial effect was observed with the regular citrus flavour composition, whereas upon addition of antimicrobial key 6 or 7, the mixture showed good efficiency in bacterial kill. Antimicrobial keys 6 and 7 proved to be the most efficient one in the present in vitro test.

Table 11

| Sample 2 | StDev | Sample 3 | StDev | Sample 4 | StDev |
|---|---|---|---|---|---|
| 0.00 | 0.15 | 1.72 | 0.25 | 1.51 | 0.22 |

Example 8

In vivo evaluation of a mouthwash containing the antimicrobial flavour composition.

Objectives were to evaluate the effectiveness of non-alcoholic mouthwash base containing 0.2 % Mint Flavours in reducing the total cultivable bacterial charge in saliva at both 1 min and 1 hour after a 1 minute wash. Two different Mint flavours were compared. These were prepared as summarized in the following table, using the same flavour base, alone or together with an antimicrobial key according to the invention.

Table 12

|  | Mint flavour 1 | Mint flavour 2 |
|---|---|---|
| Key 8 | 0 | 25 |
| MintFlavour base | 100 | 75 |

Said mint flavour base contains only flavouring ingredients that are not suitable ingredients for the antimicrobial key according to the present invention (i.e. that have a MIC above 1000 against the bacterial strains cited in the present application). Antimicrobial flavour key 8 was prepared by admixing the following ingredients in the following proportions:

Table 13

| Ingredients | Proportions (%) |
|---|---|
| 2-methylhexanoic acid | 36.2 |
| 3,4-dimethylphenol | 3.2 |
| Natactone dextro | 0.4 |
| Eugenol F | 3.2 |
| Anethole Nat | 43.0 |
| Eucalyptus globulus oil | 10.7 |
| Cinnamic aldehyde | 3.2 |

The two alcohol free mouthwashes were prepared, one with each of the mint flavour 1 and 2, by admixing the following ingredients in the following proportions:

Table 14

| Ingredients | Proportions (%) |
|---|---|
| Water (demineralised) | 82.68 |
| Sodium mono-fluorophosphate | 0.05 |
| Sodium saccharin | 0.03 |
| Sodium benzoate | 0.20 |
| Sorbitol (70% ) | 8.02 |
| Glycerin | 8.02 |
| Ethanol (95%) | 0.00 |

(continued)

| Ingredients | Proportions (%) |
|---|---|
| Cremophor® CO40 | 1.00 |
| Mint Flavor (over water) | 0.2 |

The panel was composed of 24 subjects between the age of 25 and 40. They are non-smokers, in good general health, should not have taken antibiotics 2 months prior the start of the trial. They should have no sign of oral disorders. The subjects were asked to have neither food nor drink starting at midnight the day before experiment. They were given a non-flavoured toothpaste without any Triclosan® to use the week before. They were asked to come early in the morning and rinse their mouth during one minute. Total cultivable bacterial charge in their saliva was determined before the rinse. The same measurement was done one minute and then one hour after the rinse. Saliva samples (1mL) were collected into 50ml sterile Falcon tubes. A transfer volume of $500\mu l$ saliva was stored at room temperature (RT) in Portagerm transport bottles (Biomerieux) not more than one hour before bacterial counting. Serial dilutions ($10^{-3}$ and $10^{-4}$) were done in physiological water and plate twice on Schaedler agar plate by the Spiral plater (IUL instruments) to count total anaerobic bacteria after 72 hours incubation at 37°C.

Percentage reductions in bacterial counts before and after the rinse for each of the mouthwashes were compared. One minute after the rinse, mint flavour 2 (containing the antimicrobial key of the invention) already proved to achieve a better percentage reduction in bacterial counts than the mouthwash containing mint flavour 1. This result was confirmed by the percentage reduction in bacterial counts one hour after the rinse. The mouthwash containing mint flavour 2 achieved excellent antimicrobial effect, even considerably better than one minute after the rinse. The other mouthwash was much less efficient and the percentage reduction in bacterial counts was only slightly improved after one hour.

## Claims

1. A flavouring composition comprising an antimicrobial key and optionally at least one flavouring ingredient of current use, wherein the antimicrobial key comprises 3,4-dimethylphenol together with one or more antimicrobial flavour ingredients, each having a minimum inhibitory concentration of 1000 parts per million or less against two or more strains selected from *Fusobacterium nucleatum, Fusobacterium sp., Porphyromonas gingivalis, Prevotella intermedia, Klebsiella pneumoniae, Veillonella alcalescens, Bacteroides melaninogenicus/forsythus, Selenomonas sputagena, Porphyromonas endodontalis, Prevotella melaninogenica* and *Streptococcus mutans.*

2. A flavouring composition as claimed in claim 1 wherein at least one of the antimicrobial flavouring ingredients has a minimum inhibitory concentration of less than 800 parts per million.

3. A flavouring composition according to claim 1 or claim 2 wherein at least one of the antimicrobial flavouring ingredients has a bacterial contact time BCT log count reduction of 2 or more against cariogenic bacteria *S. mutans* and/or a log count reduction of 1 or more against anaerobic pathogenic strains after 80 second contact time.

4. A flavouring composition according to any one of the preceding claims comprising from 1 to 20% by weight of the antimicrobial key, based on the total weight of the flavouring composition.

5. A flavouring composition according to any one of the preceding claims wherein the antimicrobial key comprises 3,4-dimethylphenol together with one or more ingredients selected from 3-dodecenal, acetyl cedrene, isopropyl myristate, anethole, 2-ethyl-4-(2,2,3-trimethyl-3-cyclopenten-1-yl)-2-buten-1-ol, 2-methyl-4-(2,2,3-trimethyl-3-cyclopenten-1-yl)-1-butanol, cashmerane, cedrol, cedryl acetate, cinnamic aldehyde, dimethyl acetal, cyclohexadecen-1-one, cyclopenta-decanolide, cyclopentadecanone, decen-1-ol, dihydrofamesol, dodecanal, ethylenedodecanedioate, helional, isobutyl quinoline, isocamphylcyclohexanol, isoeugenol extra, levosandol, 3-(4-tert-butylphenyl)-2-methylpropanal, menthoxypropane-1,2-diol, methyl atrarate, methyl decanal, methyl sandeflor, methyl undecanal, nerolidol, nonanol, nootkatone, 2-methylhexanoic acid, o-methoxycinnamic aldehyde, perilla alcohol, 1-(1,1,2,3,3,6-hexamethyl-5-indanyl)-1-ethanone, phenylacetaldehyde, (+)-3-methyl-5-(2,2,3-trimethyl-3-cyclopenten-1-yl)-2-pentanol, santalinol, trans-2-undecenal, undecanal and undecen-1-ol.

6. A flavouring composition according to any one of the preceding claims wherein the antimicrobial key comprises 3,4-dimethylphenol together with one or more ingredients selected from acetyl cedrene, cyclopentadecanone, nonanol,

**EP 2 099 421 B1**

isoeugenol extra, 2-methylhexanoic acid and 2-methyl-4-(2,2,3-trimethyl-3-cyclopenten-1-yl)-1-butanol.

7. A flavouring composition according to any of the preceding claims, wherein the 3,4-dimethylphenol is present in an amount of from 1 to 20% by weight, based on the total weight of the key.

8. A flavouring composition according to any of the preceding claims, wherein the flavouring composition or the anti-microbial key is encapsulated.

9. An oral care product comprising the flavouring composition according to any one of the preceding claims.

10. A beverage comprising the flavouring composition according to any one of claims 1 to 7.

11. A confectionary product comprising the flavouring composition according to any one of claims 1 to 7.


**Patentansprüche**

1. Aromazusammensetzung, umfassend eine antimikrobielle Schlüsselkomponente und gegebenenfalls mindestens einen Aromabestandteil gebräuchlicher Verwendung, wobei die antimikrobielle Schlüsselkomponente 3,4-Dimethylphenol zusammen mit einem oder mehreren antimikrobiellen Aromabestandteilen umfaßt, die jeweils eine minimale hemmende Konzentration von 1000 Teilen pro Million oder weniger gegen zwei oder mehrere Stämme, ausgewählt aus *Fusobacterium nucleatum, Fusobacterium sp., Porphyromonas gingivalis, Prevotella intermedia, Klebsiella pneumoniae, Veillonella alcalescens, Bacteroides melaninogenicus/forsythus, Selenomonas sputagena, Porphyromonas endodontalis, Prevotella melaninogenica* und *Streptococcus mutans,* haben.

2. Aromazusammensetzung nach Anspruch 1, wobei mindestens einer von den antimikrobiellen Aromabestandteilen eine minimale hemmende Konzentration von weniger als 800 Teilen pro Million hat.

3. Aromazusammensetzung gemäß Anspruch 1 oder Anspruch 2, wobei mindestens einer von den antimikrobiellen Aromabestandteilen eine logarithmische Verringerung der Zahl bei der bakteriellen Kontaktzeit BCT von 2 oder mehr gegen die kariogenen Bakterien *S. mutans* und/oder eine logarithmische Verringerung der Zahl von 1 oder mehr gegen anaerobe pathogene Stämme nach 80 Sekunden Kontaktzeit hat.

4. Aromazusammensetzung gemäß einem der vorhergehenden Ansprüche, umfassend von 1 bis 20 Gew.-% von der antimikrobiellen Schlüsselkomponente, bezogen auf das Gesamtgewicht der Aromazusammensetzung.

5. Aromazusammensetzung gemäß einem der vorhergehenden Ansprüche, wobei die antimikrobielle Schlüsselkomponente 3,4-Dimethylphenol zusammen mit einem oder mehreren Bestandteilen, ausgewählt aus 3-Dodecenal, Acetylcedren, Isopropylmyristat, Anethol, 2-Ethyl-4-(2,2,3-trimethyl-3-cyclopenten-1-yl)-2-buten-1-ol, 2-Methyl-4-(2,2,3-trimethyl-3-cyclopenten-1-yl)-1-butanol, Cashmeran, Cedrol, Cedrylacetat, Zimtaldehyd, Dimethylacetal, Cyclohexadecen-1-on, Cyclopentadecanolid, Cyclopentadecanon, Decen-1-ol, Dihydrofamesol, Dodecanal, Ethylendodecandioat, Helional, Isobutylchinolin, Isocamphylcyclohexanol, Isoeugenol extra, Levosandol, 3-(4-tert-Butylphenyl)-2-methylpropanal, Menthoxypropan-1,2-diol, Methylatrarat, Methyldecanal, Methylsandeflor, Methylundecanal, Nerolidol, Nonanol, Nootkaton, 2-Methylhexansäure, o-Methoxyzimtaldehyd, Perillaalkohol, 1-(1,1,2,3,3,6-hexamethyl-5-indanyl)-1-ethanon, Phenylacetaldehyd, (+)-3-Methyl-5-(2,2,3-trimethyl-3-cyclopenten-1-yl)-2-pentanol, Santalinol, trans-2-Undecenal, Undecanal und Undecen-1-ol, umfaßt.

6. Aromazusammensetzung gemäß einem der vorhergehenden Ansprüche, wobei die antimikrobielle Schlüsselkomponente 3,4-Dimethylphenol zusammen mit einem oder mehreren Bestandteilen, ausgewählt aus Acetylcedren, Cyclopentadecanon, Nonanol, Isoeugenol extra, 2-Methylhexansäure und 2-Methyl-4-(2,2,3-trimethyl-3-cyclopenten-1-yl)-1-butanol, umfaßt.

7. Aromazusammensetzung gemäß einem der vorhergehenden Ansprüche, wobei das 3,4-Dimethylphenol in einem Anteil von 1 bis 20 Gew.-%, bezogen auf das Gesamtgewicht der Schlüsselkomponente, vorhanden ist.

8. Aromazusammensetzung gemäß einem der vorhergehenden Ansprüche, wobei die Aromazusammensetzung oder die antimikrobielle Schlüsselkomponente eingekapselt ist.

## EP 2 099 421 B1

**9.** Mundpflegeprodukt, umfassend die Aromazusammensetzung gemäß einem der vorhergehenden Ansprüche.

**10.** Getränk, umfassend die Aromazusammensetzung gemäß einem der Ansprüche 1 bis 7.

**11.** Süßwarenprodukt, umfassend die Aromazusammensetzung gemäß einem der Ansprüche 1 bis 7.

## Revendications

**1.** Composition aromatisante comprenant une clé antimicrobienne et éventuellement au moins un ingrédient aromatisant d'usage courant, dans laquelle la clé antimicrobienne comprend du 3,4-diméthylphénol avec un ou plusieurs ingrédients aromatisants antimicrobiens ayant chacun une concentration inhibitrice minimale inférieure ou égale à 1000 parties par million vis-à-vis de deux ou plus de deux souches sélectionnées parmi *Fusobacterium nucleatum, Fusobacterium sp., Porphyromonas gingivalis, Prevotella intermedia, Klebsiella pneumoniae, Veillonella alcalescens, Bacteroides melaninogenicus/forsythus, Selenomonas sputagena, Porphyromonas endodontalis, Prevotella melaninogenica* et *Streptococcus mutans.*

**2.** Composition aromatisante selon la revendication 1 dans laquelle au moins un des ingrédients aromatisants antimicrobiens a une concentration inhibitrice minimale inférieure à 800 parties par million.

**3.** Composition aromatisante selon la revendication 1 ou la revendication 2 dans laquelle au moins un des ingrédients aromatisants antimicrobiens présente une réduction logarithmique de la population bactérienne dépendant du temps de contact (BCT, de l'anglais "Bacterial Contact Time"), de 2 ou plus vis-à-vis des bactéries cariogènes S. *mutans* et/ou une réduction logarithmique de 1 ou plus vis-à-vis des souches pathogènes anaérobies après un temps de contact de 80 secondes.

**4.** Composition aromatisante selon l'une quelconque des revendications précédentes comprenant de 1 à 20% en poids de la clé antimicrobienne, rapporté au poids total de la composition aromatisante.

**5.** Composition aromatisante selon l'une quelconque des revendications précédentes dans laquelle la clé antimicrobienne comprend du 3,4-diméthylphénol avec un ou plusieurs ingrédients sélectionnés parmi le 3-dodécénal, l'acétylcédrène, le myristate d'isopropyle, l'anéthole, le 2-éthyl-4-(2,2,3-triméthyl-3-cyclopentén-1-yl)-2-butén-1-ol, le 2-méthyl-4-(2,2,3-triméthyl-3-cyclopentén-1-yl)-1-butanol, le cashmérane, le cédrol, l'acétate de cédryle, l'aldéhyde cinnamique, le diméthylacétal, la cyclohexadécén-1-one, le cyclopentadécanolide, la cyclopentadécanone, le décén-1-ol, le dihydrofamésol, le dodécanal, l'éthylènedodécanedioate, l'hélional, l'isobutylquinoléine, l'isocamphylcyclohexanol, l'isoeugénol extra, le lévosandol, le 3-(4-tert-butylphényl)-2-méthylpropanal, le menthoxypropane-1,2-diol, l'atrarate de méthyle, le méthyl-décanal, le méthyl-sandeflor, le méthyl-undécanal, le nérolidol, le nonanol, la nootkatone, l'acide 2-méthylhexanoïque, l'aldéhyde o-méthoxycinnamique, l'alcool de périlla, la 1-(1,1,2,3,3,6-hexaméthyl-5-indanyl)-1-éthanone, le phénylacétaldéhyde, le (+)-3-méthyl-5-(2,2,3-triméthyl-3-cyclopentén-lyl)-2-pentanol, le santalinol, le trans-2-undécénal, l'undécanal et l'undécén-1-ol.

**6.** Composition aromatisante selon l'une quelconque des revendications précédentes dans laquelle la clé antimicrobienne comprend du 3,4-diméthylphénol avec un ou plusieurs ingrédients choisis parmi l'acétyl-cédrène, la cyclopentadécanone, le nonanol, l'isoeugénol extra, l'acide 2-méthylhexanoïque et le 2-méthyl-4-(2,2,3-triméthyl-3-cyclopentén-1-yl)-1-butanol.

**7.** Composition aromatisante selon l'une quelconque des revendications précédentes, dans laquelle le 3,4-diméthylphénol est présent à raison de 1 à 20% en poids, rapporté au poids total de la clé.

**8.** Composition aromatisante selon l'une quelconque des revendications précédentes, où la composition aromatisante ou la clé antimicrobienne est encapsulé(e).

**9.** Produit de soin bucal comprenant la composition aromatisante selon l'une quelconque des revendications précédentes.

**10.** Boisson comprenant la composition aromatisante selon l'une quelconque des revendications 1 à 7.

**11.** Produit de confiserie comprenant la composition aromatisante selon l'une quelconque des revendications 1 à 7.

## REFERENCES CITED IN THE DESCRIPTION

### Patent documents cited in the description

- JP 2004067530 A
- WO 2003105794 A
- WO 9951093 A
- WO 2004014348 A, Michael Gurin
- JP 2003026527 A
- EP 1238650 A, Takasago
- WO 2005104842 A
- WO 0025606 A
- WO 0117372 A
- US 4610890 A
- US 4707367 A

### Non-patent literature cited in the description

- **Mann CM ; Markham JL.** A New Method For Determining the Minimum Inhibitory Concentration of Essential Oils. *J. of Applied Microbiology,* 1998, vol. 84, 538-544
- **Shingler ; Powlowski ; Marklund.** *Journal of Bacteriology,* February 1992, 711-724
- **S. Arctander.** *Perfume and Flavour Chemicals,* 1969
- Fenaroli's Handbook of Flavour Ingredients. CRC Press, 1975
- **M.B. Jacobs.** Synthetic Food Adjuncts. van Nostrand Co., Inc, 1947